# EUROPÄISCHE PATENTSCHRIFT

(11) **EP 1 587 578 B1**
(45) Veröffentlichungstag und Bekanntmachung des Hinweises auf die Patenterteilung: **20.09.2006**
(21) Anmeldenummer: 04706136.1
(22) Anmeldetag: 29.01.2004
(51) Int. Cl.: A61N 1/36

(54) **RETINA-IMPLANTAT ZUM STIMULIEREN EINER RETINA IN ABHÄNGIGKEIT VON EINFALLENDEM LICHT**
RETINA IMPLANT FOR STIMULATING A RETINA ACCORDING TO INCIDENT LIGHT
IMPLANT RETINIEN DESTINE A STIMULER UNE RETINE EN FONCTION DE LA LUMIERE INCIDENTE

(30) Priorität: 31.01.2003 DE 10304831
(43) Veröffentlichungstag der Anmeldung: 26.10.2005
(73) Patentinhaber: Eberhard-Karls-Universität Tübingen Universitätsklinikum, 72076 Tübingen (DE)
(72) Erfinder: NISCH, Wilfried, 72070 Tübingen (DE); STELZLE, Martin, 72776 Reutlingen (DE); GRAF, Heinz-Gerhard, 71106 Magstadt (DE); ZRENNER, Eberhart, 72076 Tübingen (DE); SCHUBERT, Markus, 72074 Tübingen (DE); HÄMMERLE, Hugo, 72072 Tübingen (DE)
(74) Vertreter: Otten, Hajo
(86) Internationale Anmeldenummer: PCT/EP2004/000768
(87) Internationale Veröffentlichungsnummer: WO 2004/067088

(56) Entgegenhaltungen:
- DE-A- 19 705 988
- DE-A- 19 931 083
- GB-A- 2 370 509
- US-A- 5 895 415

## Beschreibung

Die vorliegende Erfindung betrifft ein Retina-Implantat zur Elektrostimulation einer Retina in Abhängigkeit von einfallendem Licht,
- mit einem Stimulationschip, der dazu ausgebildet ist, im Bereich der Retina eines Auges implantiert zu werden, wobei der Stimulationschip eine Vielzahl von Kontaktstellen zum Kontaktieren von Retinazellen und eine Vielzahl von lichtempfindlichen Elementen beinhaltet, die in Abhängigkeit von auftreffendem sichtbaren Licht die Kontaktstellen ansteuern, und
- mit einem Strahlungsempfänger, der abhängig von auftreffender nicht-sichtbarer Strahlung, vorzugsweise Infrarotstrahlung (IR-Strahlung), Energie für den Stimulationschip bereitstellt.

Ein solches Retina-Implantat ist bspw. aus der DE 197 05 988 A1 bekannt.

Es gibt seit einiger Zeit Bestrebungen, mit Hilfe eines Retina-Implantats der vorgenannten Art einem Verlust des Sehvermögens auf Grund von Retina-Degenerationen entgegenzuwirken. Grundgedanke ist es dabei, dem Patienten einen mikroelektronischen Stimulationschip im Bereich der degenerierten Retina zu implantieren. Der Stimulationschip besitzt eine Vielzahl von lichtempfindlichen Elementen, die in Abhängigkeit von dem einfallenden Licht elektrische Impulse im Bereich der Retina erzeugen. Das Retina-Implantat kann auf der Retina aufgesetzt sein (sogenannter epiretinaler Ansatz), oder es kann in bzw. unter der Retina implantiert sein (sogenannter subretinaler Ansatz). Die vorliegende Erfindung zielt vor allem auf die praktische Realisierung bei einem subretinalen Implantat ab, sie kann jedoch gleichermaßen auch bei einem epiretinalen Implantat oder anderen intraokularen Implantaten Anwendung finden.

Aus der EP 0 460 320 A2 ist ein subretinales Implantat bekannt, das so ausgelegt ist, dass bereits das auftreffende Umgebungslicht ausreichen soll, um die erforderlichen Stimuli für die Retinazellen zu erzeugen. Einzelheiten bezüglich einer zusätzlichen Energieversorgung des Implantats sind folglich nicht beschrieben.

In der eingangs genannten DE 197 05 988 A1 ist ein subretinales Implantat beschrieben, das mit einer für nicht-sichtbare elektromagnetische Strahlung wirksamen, fotovoltaischen Schicht versehen ist, wobei die Stimuli unter Ausnutzung der von der fotovoltaischen Schicht erzeugten Spannung lokal geschaltet werden. Dieser Ausgestaltung liegt die Idee zu Grunde, mit Hilfe von elektromagnetischer Strahlung aus dem nicht-sichtbaren Spektralbereich, nämlich Infrarotstrahlung, eine Zusatzenergie für den Stimulationschip bereitzustellen. Infolgedessen können dann auch bei schwachen Lichtverhältnissen im sichtbaren Spektralbereich hinreichend starke Stimuli erzeugt werden.

Die Schwierigkeit bei der praktischen Realisierung besteht darin, das Implantat so zu gestalten, dass es durch das Einstrahlen der Infrarotstrahlung nicht übersteuert wird, d.h. mit anderen Worten, dass es für die nicht-sichtbare Infrarotstrahlung "blind" gemacht wird. Die genannte DE 197 05 988 A1 beschreibt einen Ansatz, wonach die für die Infrarotstrahlung sensitive, fotovoltaische Schicht unterhalb einer nur schwach oder überhaupt nicht dotierten, amorphen Schicht angeordnet ist, welche für die Infrarotstrahlung durchlässig ist. Die amorphe Schicht ist andererseits sensitiv für das sichtbare Licht. Bei Dunkelheit ist die Leitfähigkeit der amorphen Schicht sehr niedrig, sie wirkt also praktisch wie ein Isolator. Fällt sichtbares Licht auf die amorphe Schicht, erhöht sich deren Leitfähigkeit um mehrere Größenordnungen. Die in der darunter liegenden fotovoltaischen Schicht mit Hilfe der Infrarotstrahlung erzeugten Ladungsträger können dann zum Stimulieren der Retinazellen durch die amorphe Schicht hindurchtreten. Die amorphe Schicht wirkt damit wie ein vom sichtbaren Licht gesteuerter, elektronischer Schalter.

In der DE 199 31 083 A1 ist ein weiteres Retina-Implantat mit zusätzlicher Energieeinkopplung beschrieben. Im Unterschied zu dem zuvor erläuterten Implantat erfolgt die Energieeinkopplung hier jedoch induktiv. Die zuvor geschilderten Schwierigkeiten bei der Einkopplung von Infrarotstrahlung entfallen damit. Andererseits erfordert die induktive Einkopplung von Zusatzenergie einen zumindest teilweise anderen Aufbau des Retina-Implantats. Bspw. wird nach dem in DE 199 31 083 A1 vorgeschlagenen Konzept eine Wandlereinrichtung benötigt, um die auf Seite des Stimulationschips induzierte Wechselspannung in eine bevorzugte Gleichspannung umzuformen. Die entsprechende Wandlereinrichtung benötigt Platz im Bereich des Retina-Implantats und ist darüber hinaus auch mit einem entsprechenden Schaltungsaufwand verbunden.

Vor diesem Hintergrund ist es eine Aufgabe der vorliegenden Erfindung, eine Alternative für ein Retina-Implantat anzugeben, bei dem Zusatzenergie mit Hilfe von elektromagnetischer Strahlung eingekoppelt werden kann.

Diese Aufgabe wird hier dadurch gelöst, dass der Strahlungsempfänger dazu ausgebildet ist, räumlich getrennt von dem Stimulationschip im Bereich des Auges implantiert zu werden, und dass der Stimulationschip Entkopplungsmittel aufweist, um nicht-sichtbare Streustrahlung von auftreffendem sichtbaren Licht zu trennen.

Anders als bei dem aus DE 197 05 988 A1 bekannten Retina-Implantat ist hiernach also vorgesehen, den Strahlungsempfänger und den eigentlichen Stimulationschip voneinander zu trennen, um so bereits eine räumliche Entkopplung der beiden zu erreichen. In einer derzeit bevorzugten Ausgestaltung ist beispielsweise vorgesehen, den Strahlungsempfänger ebenfalls im Bereich der Retina, jedoch abgesetzt vom Stimulationschip zu implantieren. Alternativ hierzu ist es jedoch auch möglich, den Strahlungsempfänger außen auf dem Auge, beispielsweise im Augenwinkel anzuordnen.

Auf Grund dieser Maßnahme ist es dann möglich, die als Zusatzenergie vorgesehene, nicht-sichtbare elektromagnetische Strahlung, vorzugsweise Infrarotstrahlung, gezielt auf den Strahlungsempfänger zu richten bzw. von den lichtempfindlichen Elementen des Stimulationschips fernzuhalten. Diese Maßnahme gibt bei der Materialauswahl und bei der konstruktiven Gestaltung der lichtempfindlichen Elemente und des Strahlungsempfängers eine größere Flexibilität und Gestaltungsbreite. Die beiden genannten Komponenten können daher jeweils besser auf ihren jeweiligen Einsatzzweck hin optimiert werden, was einen höheren Wirkungsgrad ermöglicht. Strahlt man die gewünschte Zusatzenergie bspw. mit Hilfe von IR-Laserdioden gezielt auf den räumlich abgesetzten Strahlungsempfänger, ist die unerwünschte Beeinträchtigung der lichtempfindlichen Elemente im Stimulationschip bereits wesentlich reduziert.

Praktische Versuche mit der hier vorgeschlagenen, räumlich getrennten Anordnung von Stimulationschip und Strahlungsempfänger haben jedoch gezeigt, dass die räumliche Trennung alleine nicht ausreichend ist, um ein Übersteuern der lichtempfindlichen Elemente durch die Infrarotstrahlung unter allen Bedingungen auszuschließen. Es hat sich insbesondere gezeigt, dass auf Grund von Streuungen selbst bei Verwendung von scharf bündelnden IR-Laserdioden Streustrahlungsanteile den räumlich abgesetzten Stimulationschip erreichen können. Problematisch sind dabei sowohl Streustrahlungsanteile, die den Stimulationschip "direkt" erreichen, als auch (Mehrfach-)Reflexionen der IR-Strahlung innerhalb des Auges. Infolgedessen beruht die vorliegende Erfindung auf der Idee, die räumliche Trennung von Stimulationschip und Strahlungsempfänger durch (weitere) Entkopplungsmittel auf Seiten des Stimulationschips zu ergänzen. Erst die Kombination von räumlicher Trennung und den (weiteren) Entkopplungsmitteln ermöglicht es, ein Übersteuern der lichtempfindlichen Elemente zuverlässig auszuschließen.

Das hier vorgeschlagene Retina-Implantat hat mit dem aus DE 197 05 988 A1 bekannten Retina-Implantat gemeinsam, dass Zusatzenergie mit Hilfe von nicht-sichtbarer elektromagnetischer Strahlung bereitgestellt wird, die von außen in das Auge eingestrahlt wird. Im Unterschied zu dem bekannten Retina-Implantat schlägt die vorliegende Erfindung jedoch einen anderen Weg ein, um die sich in der Praxis ergebenden Schwierigkeiten im Hinblick auf die Gefahr des Übersteuerns der lichtempfindlichen Elemente zu beseitigen. Die genannte Aufgabe ist damit vollständig gelöst.

In einer bevorzugten Ausgestaltung beinhaltet der Stimulationschip als Entkopplungsmittel ein selektives Sperrfilter, welches die nicht-sichtbare elektromagnetische Strahlung unterdrückt. Vorzugsweise ist das selektive Sperrfilter dabei als Interferenzfilter auf Basis von dielektrischen Mehrfach-Wechselschichten, insbesondere aus Titandioxid und Siliziumdioxid realisiert.

Mit dieser Ausgestaltung wird die auf Seiten des Stimulationschips nachteilige Infrarotstrahlung gezielt unterdrückt. Die Maßnahme besitzt den Vorteil, dass entsprechende Filtertechnologien sehr gut in den Produktionsprozess des Stimulationschips integriert werden können. Die Verwendung der genannten Materialien ermöglicht es zudem, die gewünschten Filtereigenschaften mit Maßnahmen zur Erzielung einer Biokompatibilität zu kombinieren, was den Herstellungsaufwand und damit die Produktionskosten reduziert.

In einer weiteren Ausgestaltung weist der Stimulationschip als Ehtkoppluhgsmittel lichtempfindliche Elemente mit einer reduzierten Empfindlichkeit für die nicht-sichtbare Strahlung auf.

Während man mit einem Sperrfilter versucht, die nicht-sichtbare Streustrahlung vom Stimulationschip fernzuhalten, beruht diese Ausgestaltung auf der Idee, das Vorhandensein unerwünschter Streuanteile der nicht-sichtbaren Strahlung "zu akzeptieren" und die lichtempfindlichen Elemente unempfindlich dagegen zu machen. Hierfür gibt es verschiedene bevorzugte Möglichkeiten, die nachfolgende näher erläutert werden. Die Maßnahme ist besonders vorteilhaft in Ergänzung zu einem selektiven Sperrfilter, da die verschiedenen zugrundeliegenden Prinzipien eine besonders wirkungsvolle Entkopplung ermöglichen. Während mit einem selektiven Sperrfilter der zuvor beschriebenen Art bspw. eine Entkopplung im Bereich von zwei bis drei Zehnerdekaden (Unterdrückung um den Faktor 1/100stel oder 1/1000stel) erreichbar ist, können mit Hilfe der nachfolgend beschriebenen Maßnahmen nochmals ein bis vier Dekaden zusätzlich erreicht werden.

In einer Ausgestaltung der zuvor genannten Maßnahme beinhalten die lichtempfindlichen Elemente eine lichtempfindliche Schicht aus einem Material, welches eine Bandlücke zwischen Valenzband und Leitungsband von mehr als 1,5 eV besitzt. Geeignete Materialien sind bspw. amorphes Silizium mit einer hohen Kohlenstoff-dotierung oder auch Galliumarsenid, wobei das amorphe Silizium auf Grund seiner geringeren Toxizität derzeit bevorzugt ist.

Die genannte Maßnahme beruht auf der Erkenntnis, dass auftreffende Infrarotstrahlung bei einer solchen Materialwahl nicht in der Lage ist, genügend viele Elektronen vom Valenzband in das Leitungsband zu befördern, weshalb die lichtempfindlichen Elemente bei dieser Materialwahl relativ unempfindlich gegenüber der Infrarotstrahlung sind. Anders ausgedrückt, tritt die Infrarotstrahlung bei dieser Materialwahl weitgehend wirkungslos durch die lichtempfindlichen Elemente hindurch. Mit dieser Maßnahme lässt sich eine Entkopplung im Bereich von etwa drei bis vier Zehnerdekaden (Faktor 1/1000stel bzw. 1/10.000stel) erreichen.

In einer weiteren Ausgestaltung weist der Stimulationschip einen schichtförmigen Aufbau mit einer oberflächennahen ersten Schicht und einer oberflächenfernen zweiten Schicht auf, wobei die oberflächennahe erste Schicht die lichtempfindlichen Elemente beinhaltet und in Richtung des einfallenden Lichts dünn ist gegenüber einer Eindringtiefe der nicht-sichtbaren Strahlung.

Diese Ausgestaltung beruht auf der Erkenntnis, dass Infrarotstrahlung auf Grund ihrer größeren Wellenlänge tiefer in ein Halbleitermaterial eindringt, als bspw. blaues oder grünes Licht aus dem sichtbaren Spektralbereich. Die hier zu Grunde liegende Idee ist folglich, die lichtempfindlichen Elemente in einem oberflächennahen Bereich des Stimulationschips anzuordnen, in dem die auftreffende Infrarot-Streustrahlung noch keine Wirkung entfaltet (noch nicht absorbiert wird). Durch diese Maßnahme lässt sich eine Entkopplung bis zu einer Zehnerdekade erreichen. Je flacher die oberflächennahe erste Schicht dabei gegenüber der Eindringtiefe der nicht-sichtbaren Strahlung ist, desto wirkungsvoller ist diese Maßnahme.

In einer weiteren Ausgestaltung weist der Stimulationschip als Entkopplungsmittel eine Taktstufe auf, weiche die lichtempfindlichen Elemente jeweils nur zeitabschnittsweise aktiviert.

Diese Maßnahme beruht auf der Idee, den Stimulationschip und den Strahlungsempfänger nicht räumlich, sondern zudem auch zeitlich voneinander zu entkoppeln. Indem man die lichtempfindlichen Elemente jeweils nur zeitabschnittsweise aktiviert, besteht die Möglichkeit, in den nicht-aktiven Zeitabschnitten Infrarotstrahlung zur Energieversorgung des Stimulationschips einzustrahlen. Mit Hilfe dieser Maßnahme lässt sich eine Entkopplung im Bereich von etwa zwei bis vier Zehnerdekaden erreichen.

In einer weiteren Ausgestaltung der Erfindung weist der Strahlungsempfänger eine für die nicht-sichtbare Strahlung selektive Antireflexionsschicht auf, und zwar vorzugsweise unmittelbar an seiner Oberseite.

Diese Maßnahme ist eine weitere wirkungsvolle Ergänzung der bereits zuvor beschriebenen Maßnahmen, da hierdurch bereits das Entstehen unerwünschter Störstrahlung im nicht-sichtbaren Spektralbereich reduziert wird. Da Streuanteile allerdings nicht nur von der Oberfläche des Strahlungsempfängers reflektiert werden, sondern bspw. auch durch Brechung an der Linse entstehen, ist diese Maßnahme in erster Linie als Ergänzung zu den vorhergehenden Maßnahmen bevorzugt. Andererseits kann nach derzeitiger Erkenntnis eine weitere Entkopplung im Bereich einer Zehnerdekade erreicht werden.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine schematische Darstellung des neuen Retina-Implantats in einem Auge,
- Fig. 2: ein erstes bevorzugtes Ausführungsbeispiel des neuen Retina-Implantats,
- Fig. 3: ein zweites bevorzugtes Ausführungsbeispiel des neuen Retina-Implantats, und
- Fig. 4: ein drittes bevorzugtes Ausführungsbeispiel des neuen Retina-Implantats.

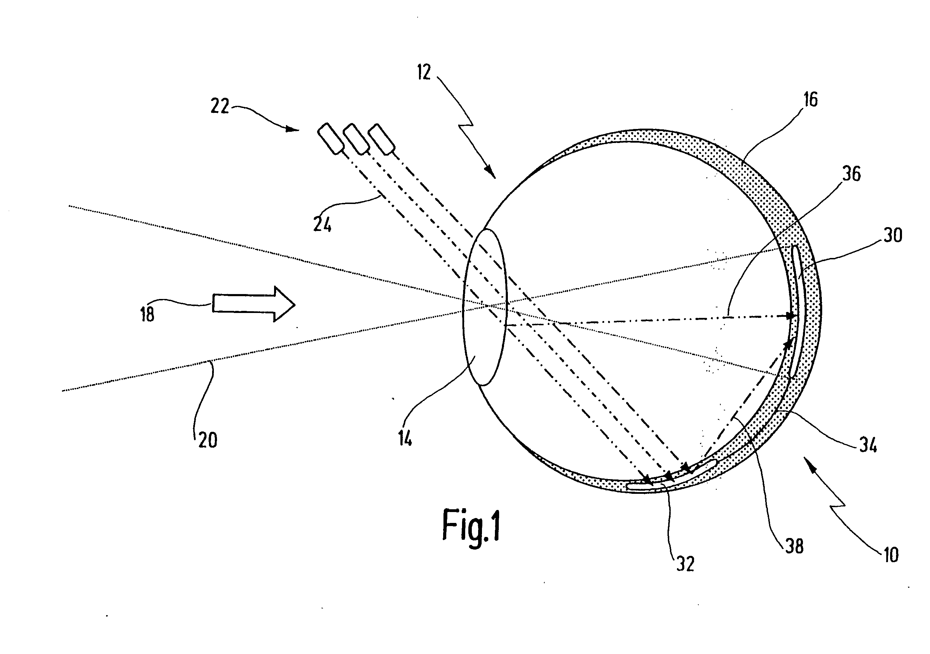

In Fig. 1 ist ein Ausführungsbeispiel des neuen Retina-Implantats in seiner Gesamtheit mit der Bezugsziffer 10 bezeichnet.

Das Retina-Implantat 10 wird in das Auge 12 eines Patienten eingepflanzt, von dem hier der Einfachheit halber nur schematisch die Linse 14 und die Retina 16 gezeigt sind. Sichtbares Licht, hier mit Hilfe eines Pfeils 18 schematisch angedeutet, fällt nach den hinlänglich bekannten optischen Gesetzen durch die Linse 14 in das Auge. Der Strahlengang des sichtbaren Lichts 18 ist hier schematisch mit der Bezugsziffer 20 gezeigt.

Mit Bezugsziffer 22 sind beispielhaft drei IR-Laserdioden bezeichnet, mit denen Energie in Form von IR-Strahlung gezielt in das Auge 12 eingekoppelt werden kann. Das Infrarotstrahlung ist mit Bezugsziffer 24 bezeichnet. Die Darstellung mit Hilfe von drei IR-Laserdioden 22 ist hier beispielhaft gewählt, um verschiedene Strahlengänge zu zeigen. In der praktischen Ausführung ist jedoch bevorzugt nur eine IR-Laserdiode 22 vorgesehen. Die Erfindung ist hierauf jedoch nicht beschränkt und kann auch mit jeder anderen Anzahl von IR-Laserdioden und auch mit anderen geeigneten Strahlungsquellen realisiert werden.

Das Retina-Implantat 10 beinhaltet in den hier gezeigten Ausführungsbeispielen jeweils den eigentlichen Stimulationschip 30, mit dessen Hilfe Retinazellen abhängig vom sichtbaren Licht 18 stimuliert werden, sowie einen davon abgesetzten Strahlungsempfänger 32. Stimulationschip 30 und Strahlungsempfänger 32 sind elektrisch miteinander verbunden, was hier durch eine Leitung 34 dargestellt ist. Der Strahlungsempfänger 32 arbeitet als Energiewandler (fotovoltaisches Element) und dient dazu, die mit Hilfe der IR-Laserdioden 22 eingestrahlte IR-Strahlung 24 aufzunehmen und in Abhängigkeit davon Energie für den Stimulationschip 30 bereitzustellen.

Durch die räumliche Trennung von Stimulationschip 30 und Strahlungsempfänger 32 sowie das gezielte Einstrahlen der IR-Strahlung auf den Strahlungsempfänger wird bereits eine wesentliche Entkopplung zwischen Stimulationschip 30 und Strahlungsempfänger 32 erreicht. Eine derartige Entkopplung ist wünschenswert, um eine Übersteuerung der im Stimulationschip 30 vorhandenen lichtempfindlichen Elemente (wird nachfolgend näher beschrieben) durch die IR-Strahlung zu verhindern. Wie sich bei praktischen Versuchen gezeigt hat, ist die räumliche Trennung allein jedoch nicht ausreichend, um unter allen denkbaren Betriebsbedingungen eine hinreichende Entkopplung zu gewährleisten. Es hat sich insbesondere gezeigt, dass beim Einstrahlen der IR-Strahlung 24 in das Auge 12 Streuanteile auftreten, die trotz der räumlichen Trennung den Stimulationschip 30 erreichen können. Beispielhaft für zwei wesentliche Streuquellen ist ein Streustrahl 36 gezeigt, der durch Brechung beim Austritt der IR-Strahlung 24 aus der Linse 14 entsteht. Ein weiterer Streustrahl 38 zeigt Reflexionen an der Oberfläche des Strahlungsempfängers 32. Darüber hinaus können innerhalb des Glaskörpers des Auges 12 Mehrfachreflexionen auftreten, so dass der Stimulationschip 30 IR-Streustrahlung aus verschiedenen Richtungen und auf Grund verschiedener Ursachen ausgesetzt ist. Erfindungsgemäß ist der Stimulationschip 30 daher mit Entkopplungsmitteln versehen, die es erlauben, die nicht-sichtbare Streustrahlung (IR-Strahlung) 24 von auftreffendem sichtbaren Licht 18 zu trennen.

Bei der nachfolgenden Beschreibung von bevorzugten Ausführungsbeispielen bezeichnen gleiche Bezugszeichen jeweils dieselben Elemente wie in Fig. 1.

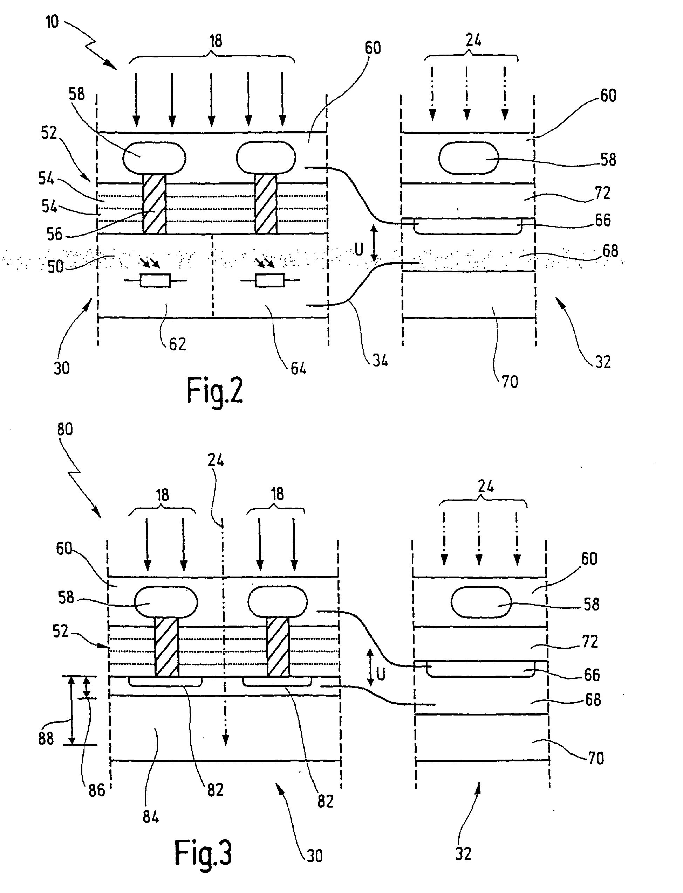

Fig. 2 zeigt ein erstes Ausführungsbeispiel des Retina-Implantats 10. Im linken Teil der Fig. 2 ist der schematische Aufbau des Stimulationschips 30 und seine Lage innerhalb der Retina 16 in einem Ausschnitt gezeigt. Der rechte Teil von Fig. 2 zeigt den schematischen Aufbau des Strahlungsempfängers 32, der räumlich abgesetzt von dem Stimulationschip 30 ebenfalls in der Retina 16 implantiert ist.

Der Stimulationschip 30 beinhaltet hier eine lichtempfindliche Schicht 50 aus einem Halbleitermaterial, welches eine Bandlücke zwischen Valenzband und Leitungsband (hier nicht dargestellt) von zumindest 1,5 eV besitzt. In einem bevorzugten Ausführungsbeispiel handelt es sich um amorphes Silizium mit einer hohen Kohlenstoff-Dotierung. Die Bandlücke beträgt in diesem bevorzugten Ausführungsbeispiel etwa 2 eV. Alternativ könnte auch Galliumarsenid verwendet sein, was auf Grund seiner toxischen Wirkung derzeit jedoch weniger bevorzugt ist, oder jedes andere Material, das die genannte Bandlücke aufweist.

Oberhalb von der lichtempfindlichen Schicht 50 ist eine Schicht 52 angeordnet, die aus mehreren Teilschichten 54 aufgebaut ist. Das Material und die Dicke der Teilschichten 54 sind so gewählt, dass die Schicht 52 als Interferenzfilter wirkt, das auftreffende Streuanteile der nicht-sichtbaren IR-Strahlung 24 gezielt unterdrückt. Bevorzugt sind die Teilschichten 54 abwechselnd aus Titandioxid und Siliziumdioxid hergestellt, da diese Materialien eine gute Bioverträglichkeit besitzen. Es können jedoch auch andere geeignete Materialien und darüber hinaus auch andere Filtertypen verwendet werden. Darüber hinaus sind auch Ausführungsbeispiele realisierbar, in denen man ohne Schicht 52 auskommt.

Mit der Bezugsziffer 56 ist eine Elektrode bezeichnet, die einen Abschnitt der lichtempfindlichen Schicht 50 mit einer zu stimulierenden Retinazelle 58 verbindet. Die Retinazelle 58 ist hier schematisch als Teil einer Gewebeschicht 60 dargestellt, die Bestandteil der Retina 16 ist.

In dem gezeigten Ausführungsbeispiel ist eine Vielzahl von Elektroden 56 vorgesehen, um eine Vielzahl von Retinazellen 58 ortsaufgelöst zu kontaktieren. Jede Elektrode 56 ist mit einem Abschnitt 62, 64 der lichtempfindlichen Schicht 50 verbunden. Die einzelnen Abschnitte 62, 64 sind damit die lichtempfindlichen Elemente, mit denen die Retinazellen 58 abhängig vom einfallenden sichtbaren Licht 18 stimuliert werden.

Die lichtempfindlichen Elemente (Abschnitte) 62, 64 sind in diesem Ausführungsbeispiel als Fotowiderstände realisiert, die ihre Leitfähigkeit bei Auftreffen von sichtbarem Licht 18 verändern. Alternativ hierzu könnten innerhalb der Schicht 50 jedoch auch Fotodioden, Fototransistoren und andere als lichtgesteuerter Schalter wirkende Elemente realisiert sein.

Der Strahlungsempfänger 32 beinhaltet in an sich bekannter Weise einen pn-Übergang, d.h. eine Struktur, die hier beispielhaft mit einer p-dotierten Schicht 66 und einer darunter liegenden n-dotierten Schicht 68 dargestellt ist. Der pn-Übergang der beiden Schichten 66, 68 ist im Hinblick auf seine Verwendung als fotovoltaisches Element optimiert (Abmessung, Dotierung der Schichten etc.). In an sich bekannter Weise ist der pn-Übergang auf einem Substrat 70 ausgebildet. Oberhalb der p-Schicht 66 ist eine für die IR-Strahlung 24 durchlässige Antireflexionsschicht 72 angeordnet. Durch diese Antireflexionsschicht 72 wird der Anteil der Streustrahlung (Streustrahl 38 in Fig. 1) verringert, was zu einer nochmals verbesserten Entkopplung des Stimulationschips 30 beiträgt.

Wie bereits erwähnt, arbeitet der pn-Übergang der Schichten 66, 68 hier als ein auf die Infrarotstrahlung 24 abgestimmtes fotovoltaisches Element. Im Bereich der Raumladungszone (hier nicht gezeigt) des pn-Übergangs entsteht bei Auftreffen der Infrarotstrahlung 24 daher eine elektrische Spannung U, die zum Stimulieren der Retinazellen 58 verwendet wird. Die erzeugte Spannung U ist dementsprechend dem Stimulationschip 30 über die Leitung 34 zugeführt.

Als ein wesentliches Entkopplungselement wirkt bei dem Stimulationschip 30 das Material der lichtempfindlichen Schicht 50. Auf Grund der hohen Bandlücke ist die relativ langwellige Infrarotstrahlung nämlich nicht in der Lage, in nennenswertem Umfang Elektronen aus dem Valenzband ins Leitungsband zu befördern. Infolgedessen verändert auftreffende IR-Streustrahlung 36, 38 die Leitfähigkeit der lichtempfindlichen Elemente 62, 64 kaum. Die lichtempfindlichen Elemente 62, 64 werden vielmehr in Abhängigkeit von auftreffendem sichtbaren Licht 18 in ihrer Leitfähigkeit gesteuert und können dementsprechend abhängig vom sichtbaren Licht die Retinazellen 58 mit der anliegenden Spannung U stimulieren.

In Fig. 3 ist ein weiteres Ausführungsbeispiel des Retina-Implantats in seiner Gesamtheit mit der Bezugsziffer 80 bezeichnet. Der Strahlungsempfänger 32 ist dabei genau so aufgebaut wie bei dem Ausführungsbeispiel gemäß Fig. 2. Ferner besitzt das Retina-Implantat 80 vorteilhafterweise wiederum ein selektives Sperrfilter, hier in Form des Interferenzfilters 52.

Im Unterschied zu dem Ausführungsbeispiel gemäß Fig. 2 besitzt der Stimulationschip 30 hier jedoch als lichtempfindliche Elemente eine Vielzahl von Fotodioden, von denen symbolisch jeweils der pn-Übergang 82 gezeigt ist. Die Fotodioden 82 sind in an sich bekannter Weise auf einem Substrat 84 ausgebildet. Als ein wesentliches Entkopplungsmittel gegenüber IR-Streustrahlung ist hier vorgesehen, dass die Dicke 86 der lichtempfindlichen pn-Übergänge 82 klein ist gegenüber der Eindringtiefe 88 der IR-Streustrahlung 24. Da die IR-Streustrahlung auf Grund ihrer vergleichsweise langen Wellenlänge tiefer in das Halbleitermaterial des Stimulationschips 30 eindringt als bspw. sichtbares Licht im blauen oder grünen Spektralbereich, kann durch die beschriebene Dimensionierung und Anordnung der lichtempfindlichen Elemente eine gute Entkopplung des Stimulationschips 30 gegenüber der IR-Streustrahlung erreicht werden.

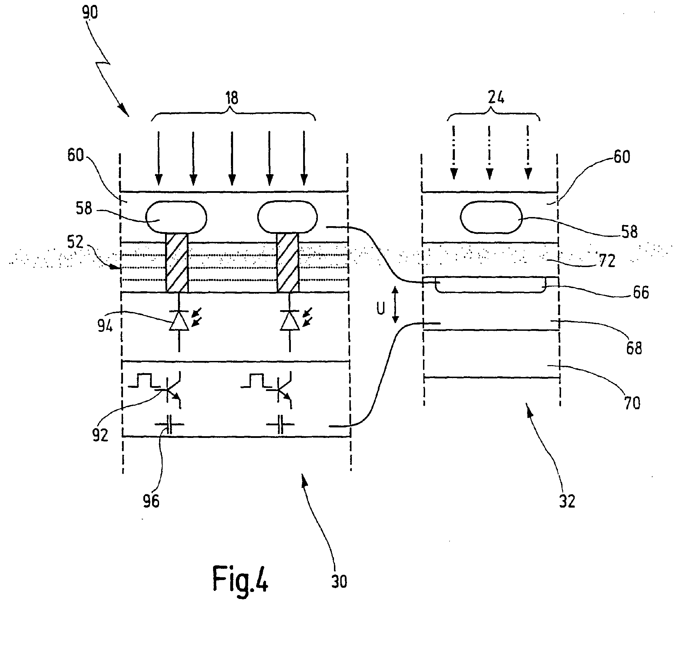

In Fig. 4 ist ein weiteres Ausführungsbeispiel des Retina-Implantats in seiner Gesamtheit mit der Bezugsziffer 90 bezeichnet. Gleiche Bezugszeichen bezeichnen dieselben Elemente wie zuvor.

Als Entkopplungsmittel besitzt der Stimulationschip 30 hier eine Vielzahl von Taktstufen 92, mit denen die lichtempfindlichen Elemente (hier Fotodioden 94) jeweils nur zeitabschnittsweise aktiviert werden. Eine solche Anordnung ermöglicht es, die IR-Strahlung 24 jeweils in den Zeitabschnitten in das Auge 12 einzukoppeln, in denen die lichtempfindlichen Elemente 94 inaktiv sind. Anders ausgedrückt, erfolgt die Einkopplung der IR-Energie in diesem Fall pulsweise, und zwar in den Zeitabschnitten, in denen die lichtempfindlichen Elemente 94 inaktiv sind. Um die eingestrahlte Energie zur Stimulation der Retinazellen 58 bereitzuhalten, sind in diesem Fall ferner Speicherelemente, hier bspw. in Form von Kondensatoren 96, vorgesehen. Darüber hinaus besitzt das Retina-Implantat 90 wiederum als weiteres Entkopplungsmittel das Interferenzfilter 52.

Es versteht sich, dass Kombinationen der hier anhand von einzelnen Ausführungsbeispielen dargestellten Entkopplungsmittel auch in hier nicht ausdrücklich gezeigter Form möglich sind, um ggf. eine noch höhere Entkopplung zu erreichen.

Experimentelle Untersuchungen an einer Modellapparatur, toten Schweineaugen sowie lebenden und toten Kaninchenaugen ergaben folgende Ergebnisse:

Um eine hinreichende Stromversorgung für den Stimulationschip 30 zu gewährleisten, muss eine optische Leistung von 15 mW auf den Strahlungsempfänger 32 eingestrahlt werden. Bei einer Anordnung wie in Fig. 1 gezeigt fällt dabei auf Grund verschiedener Reflexionen IR-Streustrahlung 36, 38 auf den Stimulationschip 30, die in der Größenordnung von 0,09 mW/cm² liegt. Dies bedeutet, dass ca. 1/2000 der eingestrahlten IR-Strahlung auf den Stimulationschip 30 fällt.

0,09 mW/cm² entspricht in etwa der Situation, wenn eine mit Tageslicht beleuchtete Fläche, etwa eine Buchseite, auf den Stimulationschip 30 abgebildet wird.

Durch die räumliche Trennung von Stimulationschip 30 und Strahlungsempfänger 32 ergibt sich also bereits eine starke Streulichtunterdrückung, die jedoch in vielen Fällen, beispielsweise bei einer Helligkeit, die geringer ist als das Tageslicht, nicht zu befriedigenden Sehergebnissen führen wird.

Durch den Einsatz eines selektiven Speerfilters 52, beispielsweise eines Kantenfilters, mit einer Antireflexionsschicht 32, kann eine weitere Streulichtunterdrückung um zwei bis drei Größenordnungen erreicht werden.

Durch eine geeignete Wahl der Bandlücke ergibt sich eine weitere Streulichtunterdrückung von ein bis zwei Größenordnungen, wobei durch die zeitliche Trennung die Streulichtunterdrückung um zumindest eine weitere Größenordnung verbessert werden kann.

## Patentansprüche

1. Retina-Implantat zum Stimulieren einer Retina (16) in Abhängigkeit von einfallendem Licht (18),
- mit einem Stimulationschip (30), der dazu ausgebildet ist, im Bereich der Retina (16) eines Auges (12) implantiert zu werden, wobei der Stimulationschip (30) eine Vielzahl von Kontaktstellen (56) zum Kontaktieren von Retinazellen (58) und eine Vielzahl von lichtempfindlichen Elementen (62, 64; 82; 94) beinhaltet, die in Abhängigkeit von auftreffendem sichtbaren Licht (18) die Kontaktstellen (56) ansteuern, und
- mit einem Strahlungsempfänger (32), der abhängig von auftreffender nicht-sichtbarer Strahlung (24), vorzugsweise IR-Strahlung, Energie für den Stimulationschip (30) bereitstellt,
**dadurch gekennzeichnet,**
- **dass** der Strahlungsempfänger (32) dazu ausgebildet ist, räumlich getrennt von dem Stimulationschip (30) im Bereich des Auges (12) implantiert zu werden, und
- **dass** der Stimulationschip (30) Entkopplungsmittel (50; 52; 86, 88; 92) aufweist, um nicht-sichtbare Streustrahlung (36, 38) von auftreffendem sichtbaren Licht (18) zu trennen.

2. Retina-Implantat nach Anspruch 1, **dadurch gekennzeichnet, dass** der Stimulationschip (30) als Entkopplungsmittel ein selektives Sperrfilter (52) beinhaltet, welches die nicht-sichtbare Strahlung unterdrückt.

3. Retina-Implantat nach Anspruch 1 oder 2, **dadurch gekennzeichnet, dass** der Stimulationschip (30) als Entkopplungsmittel lichtempfindliche Elemente (62, 64; 82) mit einer reduzierten Empfindlichkeit für die nicht-sichtbare Strahlung (24) aufweist.

4. Retina-Implantat nach Anspruch 3, **dadurch gekennzeichnet, dass** die lichtempfindlichen Elemente (62, 64) eine lichtempfindliche Schicht (50) aus einem Material beinhalten, welches eine Bandlücke zwischen Valenzband und Leitungsband von mehr als 1,5 eV besitzt.

5. Retina-Implantat nach Anspruch 3 oder 4, **dadurch gekennzeichnet, dass** der Stimulationschip (30) einen schichtförmigen Aufbau mit einer oberflächennahen ersten Schicht und einer oberflächenfernen zweiten Schicht (84) aufweist, wobei die oberflächennahe erste Schicht die lichtempfindlichen Elemente (82) beinhaltet und in Richtung des einfallenden Lichts dünn ist gegenüber einer Eindringtiefe (88) der nicht-sichtbaren Strahlung (24).

6. Retina-Implantat nach einem der Ansprüche 1 bis 5, **dadurch gekennzeichnet, dass** der Stimulationschip (30) als Entkopplungsmittel eine Taktstufe (92) aufweist, welche die lichtempfindlichen Elemente (94) jeweils nur zeitabschnittsweise aktiviert.

7. Retina-Implantat nach einem der Ansprüche 1 bis 6, **dadurch gekennzeichnet, dass** der Strahlungsempfänger (32) eine Antireflexionsschicht (72) für die nicht-sichtbare Strahlung (24) aufweist.

## Claims

1. A retina implant for stimulating a retina (16) as a function of incident light (18),
- having a stimulation chip (30) that is designed to be implanted in the area of the retina (16) of an eye (12), the stimulation chip (30) including a multiplicity of contact points (56) for making contact with retina cells (58) and a multiplicity of light-sensitive elements (62, 64; 82; 94) that drive the contact points (56) as a function of impinging visible light (18), and
- having a radiation receiver (32) that provides energy for the stimulation chip (30) as a function of impinging invisible radiation (24), preferably IR radiation,
**characterized in that**
- the radiation receiver (32) is designed so as to be implanted in an area of the eye (12) to be spatially separated from the stimulation chip (30), and
- the stimulation chip (30) has decoupling means (50; 52; 86, 88; 92) in order to separate invisible scattered radiation (36, 38) from impinging visible light (18).

2. The retina implant of claim 1, **characterized in that** the stimulation chip (30) includes as decoupling means a selective bandstop filter (52) that suppresses the invisible radiation.

3. The retina implant of claim 1 or 2, **characterized in that** the stimulation chip (30) has as decoupling means light-sensitive elements (62, 64; 82) of reduced sensitivity to the invisible radiation (24).

4. The retina implant of claim 3, **characterized in that** the light-sensitive elements (62, 64) include a light-sensitive layer (50) made from a material that has a bandgap of more than 1.5 eV between valence band and conduction band.

5. The retina implant of claim 3 or 4, **characterized in that** the stimulation chip (30) has a layered structure with a first layer near the surface and a second layer (84) remote from the surface, the first layer near the surface including the light-sensitive elements (82) and being thin in the direction of the incident light by comparison with a penetration depth (88) of the invisible radiation (24).

6. The retina implant of anyone of claims 1 to 5, **characterized in that** the stimulation chip (30) has as decoupling means a clock stage (92) that activates the respective light-sensitive elements (94) only for time periods.

7. The retina implant of anyone of claims 1 to 6, **characterized in that** the radiation receiver (32) has an antireflection layer (72) for the invisible radiation (24).

## Revendications

1. Implant rétinien destiné à stimuler une rétine (16) en fonction de la lumière incidente (18),
- comportant une puce de stimulation (30), qui est destinée à être implantée dans la zone de la rétine (16) d'un oeil (12), la puce de stimulation (30) comportant une pluralité de plages de contact (56), destinées à mettre en contact des cellules rétiniennes (58), et une pluralité d'éléments photosensibles (62, 64 ; 82 ; 94), qui activent les plages de contact (56) en fonction de la lumière visible (18) incidente, et
- comportant un récepteur de rayonnement (32) qui, en fonction du rayonnement non visible (24) incident, de préférence un rayonnement infrarouge, fournit de l'énergie pour la puce de stimulation (30),
**caractérisé en ce que**
- le récepteur de rayonnement (32) est destiné à être implanté dans la zone de l'oeil (12) en étant séparé dans l'espace de la puce de stimulation (30), et
- la puce de stimulation (30) comporte des moyens de découplage (50 ; 52 ; 86, 88 ; 92) pour séparer le rayonnement diffusé non visible (36, 38) de la lumière visible (18) incidente.

2. Implant rétinien selon la revendication 1, **caractérisé en ce que** la puce de stimulation (30) comporte un filtre d'arrêt (52) sélectif formant un moyen de découplage, lequel arrête le rayonnement non visible.

3. Implant rétinien selon la revendication 1 ou 2, **caractérisé en ce que** la puce de stimulation (30) comporte des éléments photosensibles (62, 64 ; 82) formant des moyens de découplage et ayant une sensibilité réduite pour le rayonnement non visible (24).

4. Implant rétinien selon la revendication 3, **caractérisé en ce que** les éléments photosensibles (62, 64) comportent une couche photosensible (50) qui est réalisée dans un matériau qui possède un écart énergétique de plus de 1,5 eV entre la bande de valence et la bande de conduction.

5. Implant rétinien selon la revendication 3 ou 4, **caractérisé en ce que** la puce de stimulation (30) comporte une structure en couches avec une première couche à proximité de la surface et une deuxième couche (84) éloignée de la surface, la première couche à proximité de la surface contenant les éléments photosensibles (82) et, dans la direction de la lumière incidente, est mince par rapport à une profondeur de pénétration (88) du rayonnement non visible (24).

6. Implant rétinien selon l'une quelconque des revendications 1 à 5, **caractérisé en ce que** la puce de stimulation (30) comporte un niveau de synchronisation (92), par lequel les éléments photosensibles (94) sont activés seulement par intervalles de temps.

7. Implant rétinien selon l'une quelconque des revendications 1 à 6, **caractérisé en ce que** le récepteur de rayonnement (32) comporte une couche antireflet (72) pour le rayonnement non visible (24).
